# EUROPÄISCHE PATENTSCHRIFT

(11) **EP 0 294 685 B1**
(45) Veröffentlichungstag und Bekanntmachung des Hinweises auf die Patenterteilung: **02.08.1995**
(21) Anmeldenummer: 88108661.5
(22) Anmeldetag: 31.05.1988
(51) Int. Cl.: C07D 215/04, C07D 215/18

(54) **Verfahren zur Herstellung von Chinolinen**
Process for the preparation of quinolines
Procédé de préparation de quinoléines

(30) Priorität: 06.06.1987 DE 3719014
(43) Veröffentlichungstag der Anmeldung: 14.12.1988
(73) Patentinhaber: BASF Aktiengesellschaft, 67063 Ludwigshafen (DE)
(72) Erfinder: Merkle, Hans Rupert, Dr., D-6700 Ludwigshafen (DE); Müller, Albrecht, Dr., D-6700 Ludwigshafen (DE); Reissenweber, Gernot, Dr., D-6737 Boehl-Iggelheim (DE)

(56) Entgegenhaltungen:
- US-A- 2 358 162
- US-A- 3 787 418

## Beschreibung

Die Erfindung betrifft ein Verfahren zur Herstellung von Chinolinen durch Umsetzung von Anilinen mit α,β-ungesättigten Aldehyden oder Ketonen in Schwefelsäure in Gegenwart katalytischer Mengen Iod bei erhöhter Temperatur.

Es ist bekannt, Chinoline nach Skraup durch Umsetzung von Anilinen mit Glycerin bzw. α,β-ungesättigten Aldehyden oder α,β-ungesättigten Ketonen bei Anwesenheit eines Oxidationsmittels, wie Nitrobenzol, Arsenpentoxid, Eisen-III-oxid oder Pikrinsäure, in konzentrierter Schwefelsäure herzustellen (A. Weissberger und E.C. Taylor, Heterocyclic Compounds, Vol. 32, I, S. 100 bis 117). Die dabei erzielten Ausbeuten sind mäßig. Außerdem bringt der Einsatz dieser Oxidationsmittel eine erhebliche Abwasserbelastung mit sich, so daß diese Verfahrensweise für ein technisches Verfahren ungeeignet ist.

Weiterhin ist aus GB-A-549 502 bekannt, bei der Umsetzung von Anilinen mit Glycerin in konzentrierter Schwefelsäure Iod als Oxidationsmittel einzusetzen. Auch hier sind die erzielten Ausbeuten ebenfalls nur mäßig. Von Nachteil ist ebenfalls, daß hierbei ein großer Überschuß an Glycerin erforderlich ist.

Der Erfindung lag die Aufgabe zugrunde, ein Verfahren zu entwickeln, das im großtechnischen Maßstab durchführbar ist und nach dem Chinoline in hohen Ausbeuten erhalten werden.

Demgemäß wurde ein Verfahren zur Herstellung von Chinolinen der Formel 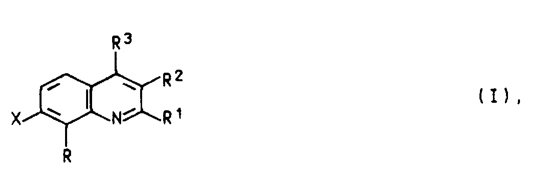
in der
- R: C₁-C₅-Alkyl,
- R¹, R², R³: Wasserstoff oder C₁-C₅-Alkyl und
- X: Wasserstoff oder Halogen bedeuten,
mit der Maßgabe, daß R auch für Wasserstoff steht, wenn X Wasserstoff bedeutet,
durch Umsetzung von Anilinen der Formel 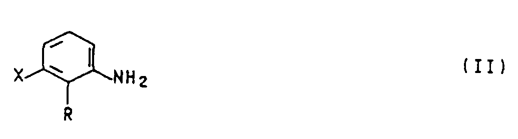
mit α,β-ungesättigten Aldehyden oder Ketonen der Formel 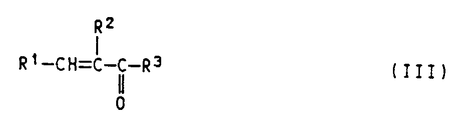
in Schwefelsäure in Gegenwart katalytischer Mengen Iod bei erhöhter Temperatur gefunden, das dadurch gekennzeichnet ist, daß die Umsetzung in Schwefelsäure von etwa 70 bis 85 Gew.% durchgeführt wird mit der Maßgabe, daß - falls die Verbindung der Formel III aus Glycerin in situ gebildetes Acrolein ist - das Molverhältnis Glycerin : Anilin der Formel II etwa 1:1 bis 1,2:1 beträgt und das bei der Bildung von Acrolein aus Glycerin entstehende Wasser während der Reaktion abdestilliert wird.

Die Umsetzung kann wie folgt ausgeführt werden:
Zu einer auf 100 bis 140°C vorgewärmten Lösung von Anilin II in Schwefelsäure und katalytischen Mengen Iod wird während 60 bis 90 Minuten Aldehyd bzw. Keton III zugefahren, wobei die Temperatur des Reaktionsgemisches möglichst nicht unter 100°C gehalten wird. Zweckmäßigerweise liegt die Temperatur im Bereich von ca. 100 bis 150°C, vorzugsweise im Bereich von 115 bis 130°C. Nach Zulaufende wird die Reaktionslösung durch Zugabe einer wäßrigen Lauge, beispielsweise Natronlauge, bei 70 bis 100°C neutralisiert, wobei sich das gewünschte Produkt als organische Phase aus der wäßrigen Phase abscheidet. Anschließend wird in an sich üblicher Weise, z.B. durch Extraktion, aufgearbeitet.

Die Konzentration der Schwefelsäure kann zwischen etwa 70 Gew.% und etwa 85 Gew.% variieren. Zweckmäßigerweise wird Schwefelsäure verwendet, die eine Konzentration von ca. 70 bis 80 Gew.% hat.

Die Ausgangsstoffe werden zweckmäßigerweise in einem Molverhältnis von etwa 1:1 bis 1:1,5, vorzugsweise etwa 1:1,1 bis 1:1,3 Anilin II zu Aldehyd bzw. Keton III eingesetzt. Die molare Menge an Schwefelsäure beträgt 2 bis 8 Mol, vorzugsweise 2,5 bis 6 Mol, pro Mol Anilin II.

Die bei der Reaktion benötigten katalytischen Mengen Iod können dem Reaktionsgemisch in Form anorganischer Iodverbindungen, z.B. als elementares Iod, als Iodwasserstoffsäure oder als Metalliodid, wie Calciumiodid oder Natriumiodid, in fester oder flüssiger Form, z.B. in Form einer wäßrigen Lösung, zugeführt werden. Die eingesetzte Menge Iod beträgt 0,1 bis 2 Mol.%, vorzugsweise 0,2 bis 1,5 Mol.%, bezogen auf Anilin II.

Es ist überraschend, daß das erfindungsgemäße Verfahren die Chinoline I in hoher Ausbeute und Reinheit liefert, da unter den drastischen Reaktionsbedingungen Nebenreaktionen der ungesättigten Ausgangsverbindungen III mit Iod zu erwarten gewesen wäre (Houben-Weyl, Methoden der org. Chemie, Bd. 5/4, Seite 530, 535).

Das erfindungsgemäße Verfahren läßt sich sehr vorteilhaft durchführen, wenn anstelle der Ausgangsverbindungen III Glycerin eingesetzt wird, aus dem der Ausgangsstoff III (R¹, R², R³ = Wasserstoff) in situ gebildet wird. Bei Verwendung von Glycerin läßt sich das erfindungsgemäße Verfahren in gleicher Weise ausführen wie beim Einsatz von Aldehyden bzw. Ketonen III. In diesem Fall ist es jedoch äußerst vorteilhaft, nach Zugabe des Glycerins bei ca. 100 bis 150°C, zweckmäßigerweise bei 130 bis 145°C, unter gleichzeitigem Abdestillieren des entstehenden Reaktionswassers nachzurühren. Es empfiehlt sich, mindestens 60 bis 70 % des entstehenden Reaktionswassers abzudestillieren. Es genügt, einige Stunden, beispielsweise zwei bis vier Stunden, lang nachzurühren. Bei dieser Verfahrensweise ist das Chinolin I mit einem Glycerinüberschuß von 1,2 Mol, vorzugsweise 1,1 Mol pro Mol Anilin II, in hoher Ausbeute zu erhalten.

Es war nicht zu erwarten, daß die Umsetzung, ausgehend von einem geringen molaren Überschuß an Glycerin, bei Verwendung von verdünnter Schwefelsäure Chinoline in hoher Ausbeute und Reinheit liefert, da aus GB-A-549 502 bekannt ist, daß das Arbeiten in nicht konzentrierter Schwefelsaure trotz hohem Glycerinüberschuß zu sehr niedrigen Ausbeuten führt. Darüber hinaus hätte die Maßnahme des Abdestillierens von Reaktionswasser, bei der über 90 % des eingesetzten Iods das Reaktionsgemisch verläßt, ebenfalls eine Verringerung der Ausbeute bzw. Verlängerung der Reaktionszeiten erwarten lassen.

Beispiele für Ausgangsverbindungen II sind Anilin, 2-Methyl-anilin, 2-Ethyl-anilin, 2-Propyl-anilin, 2-Isopropyl-anilin, 2-Butyl-anilin, 2-Pentyl-anilin, 2-tert.-Butyl-anilin, 3-Chlor-2-methyl-anilin, 3-Chlor-2-ethyl-anilin, 3-Chlor-2-propyl-anilin, 3-Chlor-2-isopropyl-anilin, 3-Chlor-2-butyl-anilin, 3-Fluor-2-methyl-anilin, 3-Fluor-2-ethyl-anilin, 3-Fluor-2-propyl-anilin, 3-Brom-2-methyl-anilin, 3-Brom-2-ethyl-anilin.

Als Ausgangsverbindungen III kommen, außer Glycerin, beispielsweise Acrolein, Methacrolein, Crotonaldehyd, Penten-2-al, Hexen-2-al, Methylvinylketon, Ethylvinylketon und Propylvinylketon in Betracht.

Die Alkylsubstituenten für R, R¹, R² und R³ in den Formeln I, II und III können unverzweigt oder verzweigt sein und sind beispielsweise Methyl, Ethyl, n-Propyl, i-Propyl, n-Butyl, sec.-Butyl, tert.-Butyl. Als Halogensubstituenten für R kommen Chlor, Brom oder Fluor in Betracht, vorzugsweise Chlor.

Die Chinoline der Formel I dienen als Zwischenprodukte für herbizide Wirkstoffe (DE-A-3 108 873; DE-A-32 33 089).

### Beispiel 1

### 7-Chlor-8-methyl-chinolin

a) Zu einer Lösung aus 625 g 80 %iger Schwefelsäure (5,0 Mol), 141,5 g (1,0 Mol) 3-Chlor-2-methyl-anilin und 2 g (0,013 Mol) Natriumiodid werden innerhalb von 60 Minuten bei 115 bis 120°C 72,8 g (1,30 Mol) Acrolein gegeben. Nach 10 Min. Nachrühren wird mit 25 %iger Natronlauge auf pH 7 bis 8 eingestellt und bei 70 bis 90°C mit 200 ml Toluol extrahiert. Nach dem Eindampfen des Toluolextraktes erhält man 164,6 g (GC: 92,9 %ig) 7-Chlor-8-methyl-chinolin; Ausbeute: 86,1 %.
b) Zu einer Lösung aus 550 g 80 %iger Schwefelsäure (4,5 Mol), 141,5 g (1,0 Mol) 3-Chlor-2-methylanilin und 1 g (0,007 Mol) Natriumiodid werden innerhalb von 90 Min. bei 140°C 101,3 g (1,1 Mol) Glycerin gegeben. Anschließend wird 2 Stunden lang bei 140 bis 145°C nachgerührt, wobei gleichzeitig 60 ml Wasser abdestilliert werden. Die Reaktionslösung wird dann mit 25 %iger Natronlauge auf pH 7 bis 8 eingestellt und bei 70 bis 90°C mit 200 ml Toluol extrahiert. Nach dem Eindampfen des Toluolextraktes verbleiben 180,0 g (GC: 94,6 % 7-Chlor-8-methyl-chinolin, 0,25 % 3-Chlor-2-methyl-anilin, Ausbeute: 95,9 %.
   Die Destillation des Rohproduktes ergibt 166 g 7-Chlor-8-methyl-chinolin in einer Reinheit von 99,2 %; Ausbeute 92,8 %; Kp₁ : 96 bis 98°C; Fp: 40 bis 42°C.

### Beispiel 2

### 7-Chlor-4,8-dimethyl-chinolin

Zu einer Lösung aus 840 g 70 %iger Schwefelsäure (6,0 Mol), 141,5 g 3-Chlor-2-methyl-anilin (1 Mol) und 4 g (0,027 Mol) Natriumiodid werden innerhalb von 60 Min. bei 115 bis 125°C 91 g (1,30 Mol) Methylvinylketon gegeben. Nach 2 Stunden Nachrühren wird, wie im Beispiel 1a) beschrieben, aufgearbeitet. Man erhält 179 g Rohprodukt. Die Destillation des Rohprodukts ergibt 167,2 g 7-Chlor-4,8-dimethyl-chinolin mit einer Reinheit von 99,5 %; Ausbeute: 86,8 %; Fp. 51°C.

### Beispiel 3

### 4-Methyl-chinolin

Zu einer Lösung aus 840 g 70 %iger Schwefelsäure (6,0 Mol), 93 g Anilin (1 Mol) und 4 g Natriumiodid (0,027 Mol) werden innerhalb von 60 Min. bei 115 bis 125°C 91 g (1,30 Mol) Methylvinylketon gegeben. Nach 10 Minuten Nachrühren wird, wie im Beispiel 1a) beschrieben, aufgearbeitet. Man erhält 146,4 g eines braunen Öls. Die Destillation des Rohprodukts ergibt 107,3 g 4-Methyl-chinolin mit einer Reinheit von 99,3 %; Ausbeute: 75 %; Kp₁: 80°C.

### Beispiel 4

### 7-Chlor-2,8-dimethyl-chinolin

Zu einer Lösung aus 840 g 70 %iger Schwefelsäure (6,0 Mol), 141,5 g (1 Mol) 3-Chlor-2-methyl-anilin und 3 g (0,02 Mol) Natriumiodid werden innerhalb von 60 Min bei 110°C 91 g (1,30 Mol) Crotonaldehyd gegeben.

Nach dem Aufarbeiten, wie im Beispiel 1a) beschrieben, erhält man 190,2 g 7-Chlor-2,8-dimethyl-chinolin. Die Destillation des Rohprodukts ergibt 146,8 g 7-Chlor-2,8-dimethyl-chinolin mit einer Reinheit von 98,8 % (GC); Ausbeute: 75,7 %; Kp₁: 102°C.

### Beispiel 5

### 7-Chlor-3,8-dimethyl-chinolin

Zu einer Lösung aus 840 g 70 %iger Schwefelsäure (6,0 Mol), 141,5 g (1 Mol) 3-Chlor-2-methyl-anilin und 4 g (0,027 Mol) Natriumiodid werden innerhalb von 60 Min. bei 120°C 91 g (1,30 Mol) Methacrolein gegeben.

Nach dem Aufarbeiten, wie in Beispiel 1a) beschrieben, erhält man 198,4 g 7-Chlor-3,8-dimethyl-chinolin. Die Destillation des Rohprodukts ergibt 180,7 g 7-Chlor-3,8-dimethyl-chinolin mit einer Reinheit von 96,1 % (GC); Ausbeute 90,7 %; Fp. 75 bis 78°C.

## Patentansprüche

1. Verfahren zur Herstellung von Chinolinen der Formel 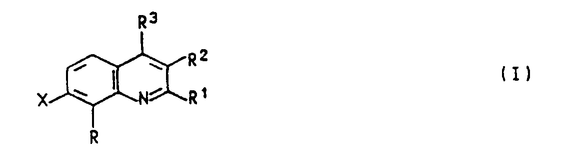 in der
R C₁-C₅-Alkyl,
R¹, R², R³ Wasserstoff oder C₁-C₅-Alkyl und
X Wasserstoff oder Halogen bedeuten,
mit der Maßgabe, daß R auch für Wasserstoff steht, wenn X Wasserstoff bedeutet,
durch Umsetzung von Anilinen der Formel 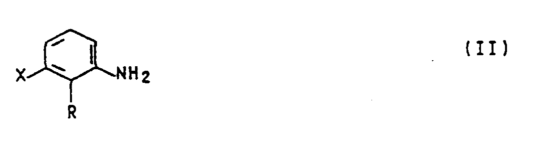 mit α,β-ungesättigten Aldehyden oder Ketonen der Formel 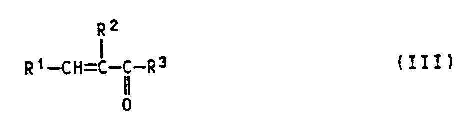 in Schwefelsäure in Gegenwart eines Oxidationsmittels bei erhöhter Temperatur, dadurch gekennzeichnet daß die Umsetzung in Schwefelsäure von etwa 70 bis 85 Gew.% in Gegenwart katalytischer Mengen Iod durchgeführt wird mit der Maßgabe, daß - falls die Verbindung der Formel III aus Glycerin in situ gebildetes Acrolein ist - das Molverhältnis Glycerin: Anilin der Formel II etwa 1:1 bis 1,2:1 beträgt und das bei der Bildung von Acrolein aus Glycerin entstehende Wasser während der Reaktion abdestilliert wird.

## Claims

1. A process for preparing quinolines of the formula 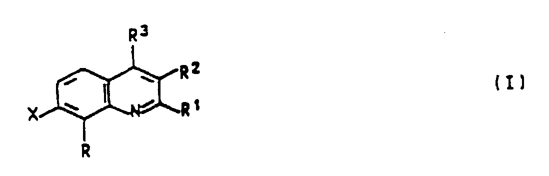 where
R is C₁-C₅-alkyl,
R¹, R² and R³ are hydrogen or C₁-C₅-alkyl and
X is hydrogen or halogen,
with the proviso that R is also hydrogen if X is hydrogen,
by reacting anilines of the formula 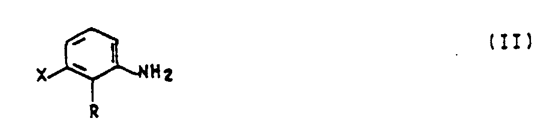 with α, β-unsaturated aldehydes or ketones of the formula 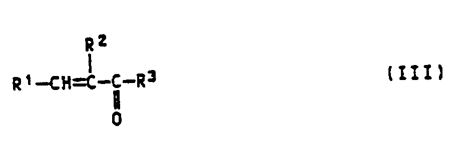 in sulfuric acid in the presence of an oxidant at elevated temperature, which comprises carrying out the reaction in from approximately 70 to 85% by weight sulphuric acid in the presence of catalytic amounts of iodine, with the proviso that, if the compound of the formula III formed from glycerol in situ is acrolein, the molar ratio glycerol:aniline of the formula II is from approximately 1:1 to 1.2:1 and the water resulting during the formation of acrolein from glycerol is distilled off during the reaction.

## Revendications

1. Procédé de préparation de quinoléines de la formule 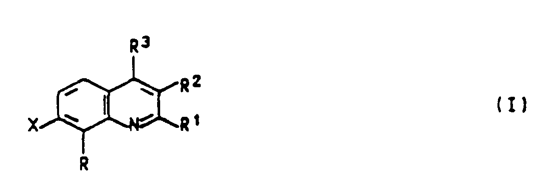 dans laquelle
R représente un radical alkyle en C₁ à C₅,
R¹, R² et R³ représentent chacun un atome d'hydrogène ou un radical alkyle en C₁ à C₅ et
X représente un atome d'hydrogène ou un atome d'halogène,
avec la condition que R représente également un atome d'hydrogène lorsque X représente un atome d'hydrogène,
par la réaction d'anilines de la formule 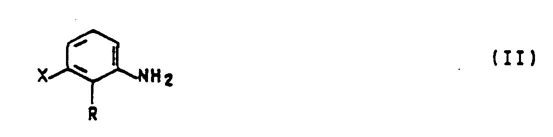 avec des cétones ou des aldéhydes α,β-insaturés de la formule 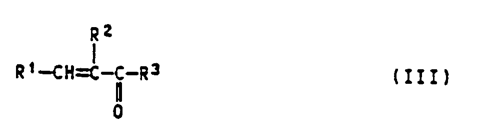 dans de l'acide sulfurique, en présence d'un agent d'oxydation et à température élevée, caractérisé en ce que l'on entreprend la réaction dans de l'acide sulfurique d'environ 70 à 85% en poids, en présence de proportions catalytiques d'iode, avec la condition que, au cas où le composé de la formule III est de l'acroléine formée in situ à partir de la glycérine - le rapport molaire glycérine:aniline de la formule II varie d'environ 1:1 à 1,2:1 et que l'eau formée au cours de la formation de l'acroléine à partir de la glycérine soit éliminée par distillation au cours de la réaction.
